# EUROPEAN PATENT APPLICATION

(11) **EP 4 406 432 A1**
(43) Date of publication of application: **31.07.2024**
(21) Application number: 21958113.9
(22) Date of filing: 19.10.2021
(51) Int. Cl.: A24F 40/46, A24F 40/42

(54) **ELECTRONIC CIGARETTE USED IN CONJUNCTION WITH MOUTH AND NOSE**

(30) Priority: 24.09.2021 CN 202111118288
(71) Applicant: Zhang, Qi, Shenzhen, Guangdong 510000 (CN)
(72) Inventor: ZHANG, Lin, Suzhou, Jiangsu 215600 (CN)
(74) Representative: JD&P Patent Attorneys Joanna Dargiewicz & Partners
(86) International application number: PCT/CN2021/124562
(87) International publication number: WO 2023/044998

(57) **Abstract**

An electronic cigarette used by cooperation of mouth and nose, including: a mouth suction electronic cigarette module and a nasal suction module. The mouth suction electronic cigarette module adopts one of a vapor electronic cigarette and a heat-not-burn electronic cigarette. The nasal suction module is arranged outside or inside of the mouth suction electronic cigarette module, and the nasal suction module contains at least one essence carrier (1501). An essence is placed in the essence carrier (1501). According to the electronic cigarette used by cooperation of mouth and nose, the essence can be partially or completely transferred from the mouth suction electronic cigarette module into the nasal suction module, so that the utilization rate and the transmission efficiency of the essence and the identification accuracy of the essence molecules reaching the olfactory nerve of the nasal cavity are improved, and the risk of the electronic cigarette caused by suction of the essence, the essence solvent, and the essence-related substance from mouth to lungs is reduced. Moreover, the types of the available essences of the electronic cigarette can be increased by means of the nasal suction module, which further helps to simplify the essence producing technology of cigarette materials such as e-liquid, solid cartridge and cigarette paste.

## Description

### Field of the Invention

The present invention relates to the field of electronic cigarette, and in particular to an electronic cigarette used by cooperation of mouth and nose.

### Description of the Related Art

Electronic cigarettes, also known as electronic atomizers, are mainly divided into two categories: vapor electronic cigarette (Vapor) and heat-not-burn electronic cigarette (HNB electronic cigarette). Comparing with traditional flue-cured tobacco, these two types of electronic cigarettes both can transmit nicotine, as well as greatly reduce the suction of tar, carbon monoxide, nitric oxide and other harmful substances into human body, so they have been used by hundreds of millions of users around the world as "harm reduction" products.

Up to now, the research on the harms of electronic cigarettes is not thorough enough, and new harms are ignored under the halo of "harm reduction". For example, the essence in e-liquid will be inhaled into lungs by mouth with the atomized aerosol, and the essence in HNB solid cartridge or cigarette paste as well as the essence being preloaded within bead set in the filter tip will also be inhaled into lungs by mouth with the atomized aerosol or HNB smoke. Due to the physiological differences of different human organs, food essences which are safe to the stomach may not be equally safe to the lungs. For example, Vitamin E Acetate is a food additive that has been used by people for a long time, however, after Vitamin E Acetate being inhaled into lungs with electronic cigarette aerosol, it will not be digested and metabolized, but covers the alveoli, causing inflammation of the lungs and even human death; butanedione and 2,3-pentanedione have been allowed adding to food as food essences for a long time, but they will be deposited in the trachea after being inhaled into lungs, causing obstruction, worsening respiratory infections and causing to "popcorn lungs".

In addition, grease, fructose, sucrose, acesulfame sweetener, aspartame sweetener, vitamins, long-chain esters, ethylene glycol, borax, coumarin, (4-ethoxyphenyl)urea, cinnamyl anthranilate, safrole, thiourea, acetoin, and the likes, are all conventional ingredients in edible essences, but once they are inhaled into lungs with the electronic cigarette aerosol or with the HNB smoke, they will seriously harm the user's health.

Nicotine/nicotine salt, propylene glycol, glycerol, and essence are the four major components of e-liquid. Sweeteners, cooling agents, and aroma substances are used to be classified as essence by people. Essences such as sweeteners, cooling agents, and aroma substances are also required adding to the solid cartridge or cigarette paste of HNB e-cigarettes. E-cigarettes containing essences are called as flavored e-cigarettes or aroma e-cigarettes. Today, essence is an indispensable key component for both vapor e-cigarettes and HNB e-cigarettes. E-cigarettes lacking essences will lose the competitiveness and cause users to choose traditional flue-cured tobacco again which produces a lot of tar. However, the types of essences are very numerous and jumbled. At present, there are more than 1000 types of essences in use in the e-cigarette industry. These essences include natural, synthetic, animal extracts and plant extracts. Along with essences, there are also essence solvents, as well as by-products in the essence production process and uncertain impurities mixed in the essence filling process, and so on.

In order to increase the market selling point, the types of essences used in e-cigarettes tend to increase, and the types of essence solvents and essence-related substances also tend to increase. The heating temperature of vapor e-cigarette e-liquid is 180-280 °C, and the heating temperature of HNB e-cigarette solid cartridge or cigarette paste is 250-380 °C, wherein the essences, essence solvents and essence-related substances within these cigarette materials have a risk of decomposing into formaldehyde, acrolein, acetone, and other hazardous substances at these high temperatures.

Currently, users employ e-cigarettes by two inhaling methods: "small circulation through mouth" and "large circulation through lungs". Specifically, the essence molecules within the atomized aerosol or HNB smoke inhaled by mouth go through the small circulation of "mouth mucosa" or the large circulation of "mouth mucosa-throat-trachea-bronchus-lungs-bronchus-trachea-throat-mouth mucosa", and then exhaled out to air from the nasal cavity or mouth, and till then essence can be sensed by the olfactory nerves in the nasal cavity, however, not only is there a loss of the number of essence molecules, but also they will be diluted and interfered by the aerosol or other substances in the surroundings, resulting in user's complaining that the e-cigarettes are poor flavors reproduction level, which in turn cause manufacturers adding more essences in the e-liquid and HNB solid cartridge or cigarette paste.

At present, more and more essences, essence solvents and essence-related substances in e-liquid or HNB solid cartridge and cigarette paste are inhaled into the lungs from mouth, and people can not conduct the pathological, toxicological research and scientific judgment of their hazards in time, often causing a lag in the safety precautions. For example, both Vitamin E Acetate and butanedione caused deaths in 2019 till people discovered their harm to lungs and began to ban them in e-cigarettes. However, a large number of essences that having not been scientifically determined to be safe for lungs are still being used in the e-cigarette industry, which undoubtedly increases the risk for users.

In addition, propylene glycol and glycerol are conventional organic solvents for essences in e-liquid, and these two accounts for about 90% by mass in e-liquid. Wherein, propylene glycol in e-cigarettes is usually derived from chemical industries such as coal and petroleum rather than plant extraction, and scientific research has confirmed that propylene glycol causes serious harm to the mouth cavity of e-cigarette users. Although glycerol is said deriving from plants, there is still in the absence of scientific and systematic research of pathology and toxicity to determine whether it is still safe after entering into the lungs from mouth and depositing in the alveoli for a long term.

Patent No. CN201310228824.X disclosed a snuff-type electronic cigarette, which is a single-channel method to inhale the smoke fog by nose completely replacing mouth, abandoning the mouth as a suction channel of e-cigarettes, and inhaling "mixed smoke" generated from "heated smoke materials" entirely through nose. In addition, the inhaled "smoke" is "mixed", indicating that there are not only essences but also other smoke substances that easily interfere with the perception of nasal olfactory nerves, such as nicotine and nicotine salts with pungent odors, as well as a large amount of glycerol are also present to produce the smoking effect, which need to be improved.

### Contents of the Invention

The main technical problem to be solved by the present invention is: to maintain the aroma felt by the user when using the e-cigarette, while reducing the amount of essences, essence solvents, and essence-related substances that enter the user's lungs.

In order to solve the above problems, the present invention adopts a technical solution: to provide an e-cigarette used by cooperation of mouth and nose, including: a mouth suction e-cigarette module and a nasal suction module, the mouth suction e-cigarette module adopts one of a vapor e-cigarette and a heat-not-burn e-cigarette, the nasal suction module is arranged outside or inside of the mouth suction e-cigarette module, and the nasal suction module contains at least one essence carrier, and essence is placed in the essence carrier.

The e-cigarette still retains the mouth transmission channel by the mouth suction e-cigarette module to inhale the atomized aerosol of e-liquid or the smoke of HNB solid cartridge or cigarette paste, and the additional nasal suction module is used as the transmission channel for the essence aroma to reach the user nose directly, whereby the e-cigarette being used for suction by the dual channels of mouth and nose at the same time. The essences previously added to the e-liquid and solid cartridge or cigarette paste in the mouth suction e-cigarette module are partially transferred or completely transferred to the nasal suction module, and the essences in the nasal suction module release volatiles at room temperature as well as being slightly heated, whereby the essence molecules in the nasal suction module can be directly transmitted to the user nasal cavity without entering the mouth cavity.

In a preferred embodiment of the present invention, the vapor e-cigarette adopts one of a high-power e-cigarette, a low-power e-cigarette, a CBD factions electronic cigarette, an open type e-cigarette, a closed type e-cigarette, an ultrasonic atomized e-cigarette, a disposable vapor e-cigarette, a cartridge-changing vapor e-cigarette, and the heat-not-burn e-cigarette adopts one of a HNB e-cigarette that consuming solid cartridge and a HNB e-cigarette that consuming cigarette paste.

So far the above-mentioned vapor e-cigarettes and heat-not-burn e-cigarettes have only a mouth suction e-cigarette module, and components of the mouth suction e-cigarette module can be categorized into four sub-modules: a suction nozzle module, an atomizing or heating module, a circuit module and a display module, wherein:
The suction nozzle module contains components such as nozzles or filters tip, and the likes;
The atomizing or heating module is further divided into: (1)e-liquid resistance atomization module, containing empty atomizing cartridge, e-liquid storage tank, e-liquid, cotton atomizing core or ceramic atomizing core, aerosol channel, the atomizing cartridge base with magnetic column and electrode tip, and other components, (2)ultrasonic atomization module or piezoelectric ceramic atomization module, (3)HNB resistance heating module, containing heating element or heating rod, heating tube, heating pot, and the likes, heater base with an electrical connection port, cartridge, cartridge sleeve, cartridge base and other components;
The circuit module contains components such as printed circuit board (PCB), temperature coefficient of resistance (TCR) thermostat, starter, temperature sensor, indicator light, switch and gear controller, battery, wire interface, wires, and the likes;
The display module contains components such as data collector and display screen, and the likes. The display module is not a necessary module for e-cigarette, but some control components can further integrated into the display module, such as control components that can adjust the output power.

The present invention provides a nasal suction module on the basis of the mouth suction e-cigarette module, and the nasal suction module may or may not be connected with the circuit module and the display module in the above mentioned four sub-modules of the mouth suction e-cigarette module.

In a preferred embodiment of the present invention, the essences include but not limited to a combination of one or more of fruit peel, fruit, fruit essence, cooling agent essence, sweetener essence, tobacco essence, meat essence, wine essence, food essence, plant herbal essence, animal spice essence, biosynthetic essence and microbial fermentation essence.

Specifically, the cooling agent essence such as menthol essence, WS-23 (N-ethyl-L-menthyl formamide) essence, WS-3 (peppermint amide) essence and the likes; the sweetener essence such as sucralose essence, neotame essence, and the likes; the fruit essence such as linalool essence, β-damascenone essence, passion fruit essence, and the likes; the tobacco essence such as tobacco extracts and the likes; the meat essence such as animal fats, artificial ethyl maltol, and the likes; the food essence such as mung bean essence, and the likes; the wine essence such as ethyl caproate, and the likes; the plant herbal essence such as russianolive flower essence, rose essence or flos sophorae essences, and the likes; the animal spice essence such as musk, civet, castoreum and ambergris, and the likes; the biosynthetic essence such as vanillin and 2-phenylethanol synthesized by biotransformation and the likes; the microbial fermentation essence such as acid essences, alcohol essences, ketone essences, lactone essences, and the likes. A small amount of solvent that comes with the essence, such as water, propylene glycol, medium-chain triglycerides, caprylic triglycerides, and the likes, can also be added to the nasal suction module together with the essence.

In a preferred embodiment of the present invention, it further includes a component retainer for fixing the nasal suction module on the outside or inside of the mouth suction e-cigarette module, wherein the component retainer includes but not limited to components such as double-sided adhesive tape, gummed tape, glue, string, clamp, snap hook, magnet, screw, and fixing ring.

In a preferred embodiment of the present invention, the essence carrier is a combination of one or more of essence bottle, essence pot, electric heating plate and electric heating pot. The electric heating plate adopts a miniature mesh electric heating plate or a non-mesh electric heating plate with integrated resistive heating component.

In addition, the height of the essence bottle cap, essence pot spout plug and electric heating pot sealing cover that accompanying the essence carrier may be increased appropriately, so as to open or close these essence carriers from the corresponding cover hole outside of the chamber cover.

In addition, a push-type switch is set over the chamber cover so as to control the volatiles outlet valve of the essence pot airtight cover, and the user can directly control the release of the essence aroma from outside of the essence pot without opening the chamber cover to remove the essence pot airtight cover.

In a preferred embodiment of the present invention, a first battery is arranged in the mouth suction e-cigarette module, and the nasal suction module may share the first battery with the mouth suction e-cigarette module, or the nasal suction module may contain a second batter, or the nasal suction module may be equipped without battery. The second battery may be a rechargeable battery or a disposable battery, and when the second battery is a rechargeable battery, a corresponding charging port needs to be provided on the e-cigarette.

In a preferred embodiment of the present invention, the nasal suction module further contains an electric heater controller, and the first battery or the second battery is connected with the electric heater controller for power supply, and the electric heater controller is connected with the heating component in the essence carrier for heating control.

In a preferred embodiment of the present invention, the nasal suction module further contains a fan controller and a fan corresponding to the essence carrier, and the first battery or the second battery is connected with the fan controller for power supply, and the fan controller is connected with the fan for operation control. The electric heater controller and the fan controller can be integrated on the electric circuit board of the mouth suction e-cigarette module or the nasal suction module, or respectively adopts conventional electrical switch such as miniature knob switch, push-type switch, inductive switch, and the likes, which can be used to control both the operation and stopping of these electrical appliances, and to adjust their operating gears. In order to facilitate the user to operate from the outside of the e-cigarette rod, these controllers can be completely or partially set on the chamber cover through the alignment holes.

In addition, in a few special cases, in addition to connecting with the fan and the electric heater in the nasal suction module, the second battery may further connect with some electrical appliances and electronic components of the mouth suction e-cigarette module, for example PCB or starter or sensor, and the likes, and when the second battery connects with all electrical appliances and electronic components in the mouth suction e-cigarette module, the second battery is equivalent to the first battery.

In a preferred embodiment of the present invention, a chamber that corresponding to the nasal suction module is provided in the shell of the mouth suction e-cigarette module, and the nasal suction module is implanted into the chamber:
Wherein, the nasal suction module can be arranged inside of the e-cigarette rod closing to the cartridge in the mouth suction e-cigarette module. In prior art, in order to connect the cartridge of the vapor e-cigarette or the cartridge of HNB e-cigarette, a chamber is provided in head of the e-cigarette rod to facilitate the insertion of the cartridge, but there is no remaining space in the chamber after the cartridge being inserted. In order to implant the nasal suction module, it is necessary to extend the shell of the e-cigarette rod in the direction of the cartridge and lengthen the connection chamber by 10-100 mm, and then move the vapor e-cigarette base with magnetic columns and electrode tips forward accordingly or move the HNB e-cigarette base with heating element and wires forward accordingly to connect with the cartridge respectively, thereby creating a chamber space for the nasal suction module with a length of 10-100 mm and almost the same height and width as those of the e-cigarette rod. It can be operated from the horizontal direction of the e-cigarette rod to sequentially implant and fix components of the nasal suction module such as essence carrier, fan, electric heater, battery and the likes into the chamber, and the alignment holes for the controllers of such as the fan and the electric heater are set in the chamber shell to facilitate control from the outside; a chamber cover can further be made on the e-cigarette rod shell, operating from the vertical direction of the e-cigarette rod to sequentially implant and fix components of the nasal suction module such as essence carrier, fan, electric heater, battery, and the likes into the chamber, the alignment cover holes for the controllers of such as the fan and the electric heater are arranged on the chamber cover to facilitate control from the outside, and the chamber cover can seal the chamber through the slot, so as to maintain a consistent appearance of the outer shell of the e-cigarette rod.

Wherein, the nasal suction module may also be set in the middle of the e-cigarette rod. Firstly, the ordinary vapor e-cigarette rod and HNB e-cigarette rod are lengthened by 10-100 mm, and then the corresponding components of the original atomizing or heating module, the circuit module and the display module are adjusted to be close to the ends of the e-cigarette rod, and a chamber for the nasal suction module with a length of 10-100 mm and almost the same height and width as those of the e-cigarette rod is created in the middle of the e-cigarette rod. The adjustment of the position of the internal components of the e-cigarette rod is a task that can be carried out by those skilled in the art without creative effort. After opening the chamber cover, operating from the vertical direction of the e-cigarette rod to implant and fix the essence carrier, fan, electric heater, battery and other components of the nasal suction module into the chamber in sequence, and the alignment cover holes for the controllers of such as the fan and the electric heater are provided on the chamber cover to facilitate control from the outside.

Wherein, the fan in the nasal suction module can be a miniature floor-standing electric fan with an integrated electric motor, or a disc-type or desktop electric fan, and so on.

In a preferred embodiment of the present invention, the nasal suction module further contains a volatiles conduit, which can be composed as a volatiles conduit assembly by connecting a volatiles conduit interface and an conduit extension tube or the likes to facilitate the lengthening of the volatiles conduit, and one end of the volatiles conduit assembly is close to the essence carrier or directly or indirectly connected with the essence carrier. Components of the volatiles conduit assembly are connected or disassembled through corresponding threads, sockets and other structures, and the volatiles conduit may be a stereotyped single tube, a plug-in multi-section tube, a telescopic sleeve type tube or a flexible universal tube, and the volatiles conduit can further be made with the appearance of elephant nose, cartoon nose, animal, plant, round hole, square, doll and trademark, and the likes, to increase the aesthetics and publicity of the volatiles conduit assembly as well as the e-cigarette.

The technical principle of the present invention is: both mouth and nose are the important exhalation and suction channels of the human body, which can work independently or cooperatively, and the cooperative work can be performed simultaneously and synchronously. Essences are mostly organic small molecules with molecular weights less than 300, such as hydrocarbons, alkenes, alcohols, aldehydes, ethers, ketones, acids, phenols, esters, terpenes, sulfur-containing compounds, nitrogen-containing compounds, and the likes, which are easy to volatilize naturally, and heating and fanning can increase the speed of volatilization of the essences. The human body's olfactory nerves to aroma are mainly concentrated in the nasal cavity. The human body's olfactory perception mechanism can be briefly described as follows: the aroma is identified by the "receptor" of the epithelial tissue of the nasal cavity and generates a "mirror image" nerve signal transmitting to the brain. The "mirror image" nerve signals generated from different aromas are differentiated, so the brain can identify specific aroma. The olfactory system is characterized by its own nerves directly connected to the brain, without the need to go through the transduction to the central nervous system and then to the brain, so, "aroma-nasal-brain" is the fastest olfactory identification channel by the unique directly connection. The present patent provides a nasal suction module on the e-cigarette, so that when the atomized aerosol and smoke generated from the mouth suction e-cigarette module being inhaled by the mouth channel, at the same time, the user's nasal channel directly inhales the essence aroma released from the nasal suction module, improving the transmission efficiency of the aroma molecules to the nasal cavity. Moreover, there are few interfering substances when the essence molecules enter the nasal cavity from the nasal suction module, which helps the essence to be accurately identified by the olfactory nerve and improves the flavor reproduction level of e-cigarettes. In addition, the human nose does not have the swallowing function as that of the mouth. The aroma, essence solvent and essence-related substances will be blocked by the nasal hairs and nasal mucosa after entering the nasal cavity, and it is difficult for them to enter the user's lungs, thereby improving the using safety of essences in e-cigarettes.

The essence in the nasal suction module volatilizes naturally at room temperature, as well as quickly volatilize by slightly heating. The slightly heating temperature is usually lower than the atomization temperature of e-liquid and the heating temperature of HNB solid cartridge or cigarette paste. Inhaling the essence aroma in the nasal suction module at a relatively low temperature through nose can not only maintain the physiological activity of essence molecules, but also reduce the risk of formaldehyde and other harmful substances from the high temperature decomposition of essences, essence solvents and essence-related substances.

To the inventor's surprise: after the essence being partially transferred or completely transferred from the mouth e-cigarette module into the nasal suction module, the flavor reproduction level of the e-cigarette will be significantly improved. The reason is analyzed that: there are only essence related substances in the nasal suction module, and the essence aroma is directly transmitted to the nasal cavity, so, when the essence molecules are perceived by the olfactory nerves of user's nose, there are relatively few other interfering substances in the surroundings, and therefore the essence aroma characteristics can be accurately identified, whereby the flavor reproduction level of the e-cigarette can be improved.

To the inventor's surprise: some e-liquids become clearer and more transparent than before after the essence being partially transferred or completely transferred from the e-liquid into the nasal suction module. The reason is analyzed that: many essences in e-liquid are derived from plant pressing or organic solvent extraction, which contain terpene essences. Although the terpene essences aroma are strong and distinctive to enhance the flavor characteristics of the vapor e-cigarettes, the solubilities of these terpene essence molecules in e-liquid are very poor, and they are easy to be separated out and cause the e-liquid turbid or even layered slicks and other serious appearance problems, compelling people to add more solvents or other new solvents to dissolve or dilute the terpene essences in such type e-liquid, which not only reduce the aroma concentration of essences, but also increase the risk of lungs for these essence solvents. In the present invention, those insoluble essences can be by partially or completely transferred into the nasal inhalation module, whereby it not only reduces the risk of the essences and essence solvents entering the lungs from mouth, but also improving the appearance of the e-liquid by making it clearer and more transparent than before.

To the inventor's surprise: after the essence being partially transferred or completely transferred from the mouth suction e-cigarette module into the nasal suction module, leading to the decrease of the essence-related substances that directly contacted with the vapor atomizing core or HNB heating element at high temperature, and the troubles of the burnt atomizing core and the carbon deposition on the atomizing core or on the heating element, which have plagued the users of e-cigarette for a long time, are significantly alleviated.

To the inventor's surprise: after the essence being partially transferred or completely transferred from the mouth suction e-cigarette module into the nasal suction module, due to the reduction of the risk of essence-related substances entering the lungs from mouth, the types of essences available for e-cigarette can be increased through the nasal suction module. For example, meat essences or wine essences and the likes can be used for nasal suction module, and even thick cigarette paste essences of tobacco with a tobacco-tar-like odor can be used, and it is difficult to use these types of essences in the prior arts of e-cigarettes due to problems such as poor appearance, poor transparency and difficulties of blending essences to be matched. In addition, transferring out the essence from the mouth suction e-cigarette module may also simplify the essences modulation technology, as well as simplifying the production processes of cigarette materials such as e-liquids, solid cartridges and cigarette pastes.

The differences between the present invention and the prior arts of e-cigarette and snuff are as follows:
(1)The present invention uses the nose channel to inhale the essence of the nasal suction module, wherein the essence can be inhaled into nose at room temperature, as well as with slightly heating. However, in the prior arts of e-cigarettes, the essence substances need to be heated up to 180-380 °C with e-liquid and solid cartridge or cigarette paste before they can be inhaled into the human body by the mouth suction e-cigarette module, which has a risk of deterioration at high temperatures, and the present invention helps to maintain the original flavor and biological activity of the essence;
(2)In the prior art, the essence of e-cigarette is inhaled by mouth along with the atomized aerosol of the cartridge, and it needs to go through a small circulation of "mouth mucosa" or a large circulation of "mouth mucosa-throat-trachea-bronchus-lungs-bronchus-trachea-throat-mouth mucosa" and then being exhaled out from the nasal cavity or mouth, till then, being perceived by olfactory nerves in the nasal cavity. The essence from the nasal suction module in the e-cigarette of the present invention will be directly inhaled by the nasal cavity and quickly perceived by the olfactory nerves, which improves the transmission efficiency of the essence molecules to the nasal cavity and the accuracy of identification;
(3)Users inhale atomized aerosols or HNB smoke only through the mouth in the prior art of e-cigarettes, and users suck snuff powder only through the nose in the prior art of snuff, both are single inhalation channel. However, the present invention discloses an e-cigarette with dual inhalation channels that can be used by cooperation of mouth and nose simultaneously.
(4)The crushed solid particles of tobacco leaves, lime powder and other substances are directly inhaled into the nasal cavity by snuff, which are easy to damage the nasal mucosa and olfactory nerves, and are very harmful to the nose. However, it is essence aroma that directly inhaled into the nasal cavity from the nasal suction module in the e-cigarette of the present invention, which is soft, comfortable, and little harm to the nose;
(5)The CN201310228824.X patent completely abandoned the mouth suction channel, and what directly inhaled by the human nose is "smoke fog", with characteristics of "smoke" and "fog". However, there are dual suction channels that can be used by cooperation of mouth and nose in the present invention, and it is the essence aroma that directly inhaled into the nasal cavity from the nasal suction module of the e-cigarette, which is not smoky, so there are obvious differences in the inhaled substances and visual effects between the CN201310228824.X and the present invention.

The beneficial effects of the present invention are: an e-cigarette used by cooperation of mouth and nose is disclosed by the present invention, wherein the essence substances of the e-liquid, solid cartridge or cigarette paste can be partially or completely transferred from the mouth suction e-cigarette module into the nasal inhalation module. While the mouth inhaling the atomized aerosol or smoke of e-cigarette, and at the same time, the essence molecules in the nasal suction module can be directly inhaled into nasal cavity by the nose, which improving the utilization rate of the essence, as well as the transmission efficiency and identification accuracy of the essence molecules to the olfactory nerve in the nasal cavity. The risk of inhaling essence substances, such as essences, essence solvents, essence by-products, and uncertain impurities in the essence production process and the likes from mouth into the lungs by using e-cigarette is decreased, and the troubles of burnt atomizing core and the carbon deposition on atomizing core or on heating element are alleviated, and the types of essences available for e-cigarettes can be increased through the nasal suction module, and simplifying the producing technology of essences in cigarette materials such as e-liquids, solid cartridges and cigarette pastes.

### Brief Description of the Drawings

In order to more clearly illustrate the technical solutions in the embodiments of the present invention, the accompanying drawings that need to be used in the description of the embodiments will be briefly described below, and it is obvious that the accompanying drawings in the following description are only some of the embodiments of the present invention, and other drawings can be obtained by those skilled in the art based on these accompanying drawings without creative work, wherein:

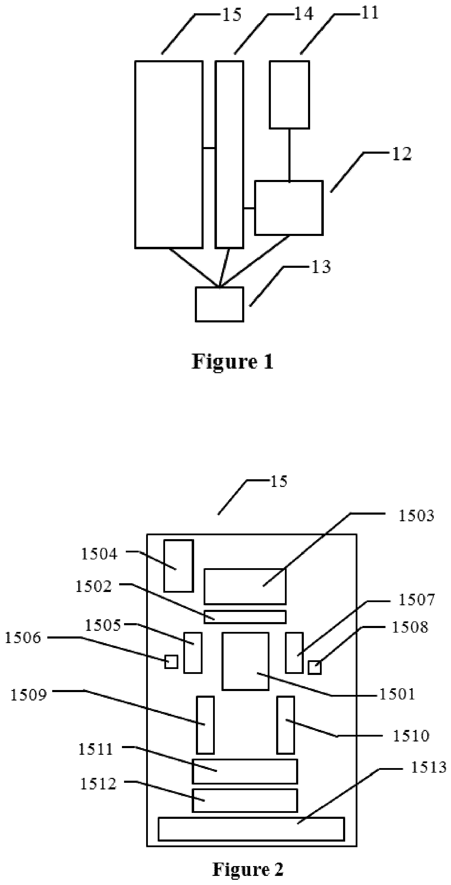

Figure 1 is a structural frame diagram of an e-cigarette containing a nasal suction module, and the components are as follows: 11 suction nozzle module, 12 atomizing or heating module, 13 circuit module, 14 display module, 15 nasal suction module;
Figure 2 is the frame diagram of the component structure of nasal suction module (15), 1501 essence carrier, 1502 essence carrier cover, 1503 essence volatiles conduit, 1504 conduit extension tube, 1505 fan, 1506 fan controller, 1507 electric heater, 1508 electric heater controller, 1509 fan circuit, 1510 electric heater circuit, 1511 battery terminal wire interface, 1512 battery and 1513 component retainer;

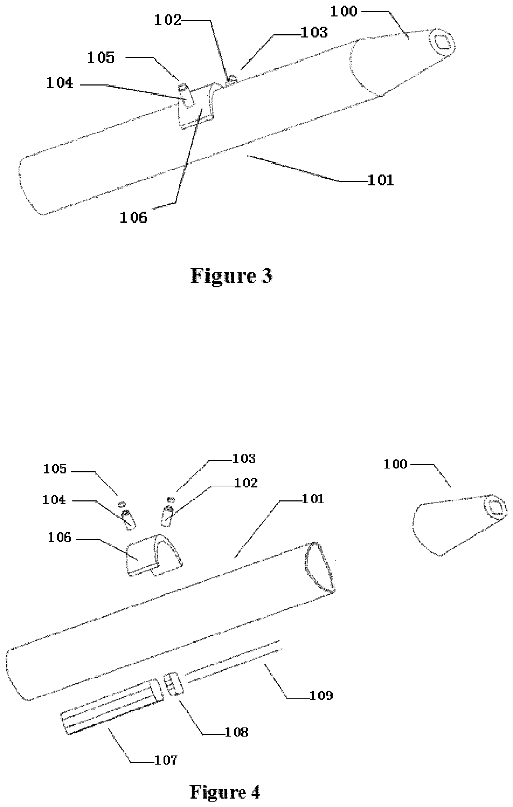

Figure 3 is an external view of the e-cigarette in the Embodiment 1, and Figure 4 is an exploded view of the structure of the e-cigarette in the Embodiment 1, and the components are as follows: 100 suction nozzle, 101 e-cigarette set, 102 first essence bottle, 103 first essence bottle cap, 104 second essence bottle, 105 second essence bottle cap, 106 double-sided adhesive tape, 107 the first battery, 108 the first battery terminal wire interface, 109 atomizer circuit;

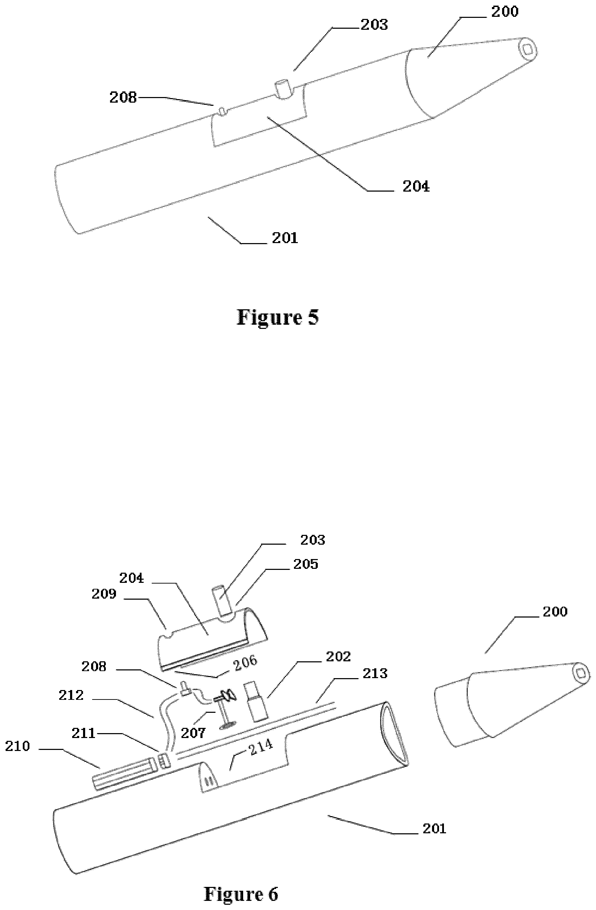

Figure 5 is the external view of the e-cigarette in the Embodiment 2, and Figure 6 is an exploded view of the structure of the e-cigarette in the Embodiment 2, and the components are as follows: 200 atomizing cartridge, 201 cigarette rod, 202 essence bottle, 203 essence bottle cap, 204 chamber cover, 205 essence bottle cap alignment cover hole, 206 chamber cover slot, 207 fan, 208 fan knob switch, 209 fan knob switch alignment cover hole, 210 the first battery, 211 the first battery terminal wire interface, 212 wires, 213 atomizer circuit, 214 chamber;

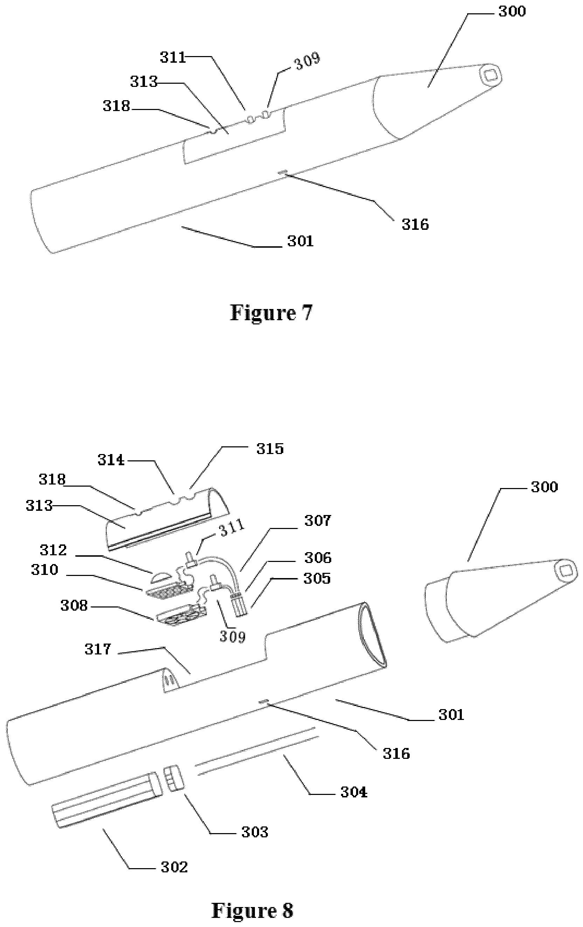

Figure 7 is an external view of the e-cigarette in the Embodiment 3, and Figure 8 is an exploded view of the structure of the e-cigarette in the Embodiment 3, and the components are as follows: 300 atomizing cartridge, 301 cigarette rod, 302 the first battery, 303 the first battery terminal wire interface, 304 atomizer circuit, 305 the second battery, 306 the second battery terminal wire interface, 307 wires, 308 fan, 309 fan controller, 310 mesh electric heating plate, 311 electric heating plate controller, 312 solid essence, 313 chamber cover, 314 electric heating plate controller cover hole, 315 fan controller cover hole, 316 the second battery charging port, 317 chamber, 318 essence aroma outlet;

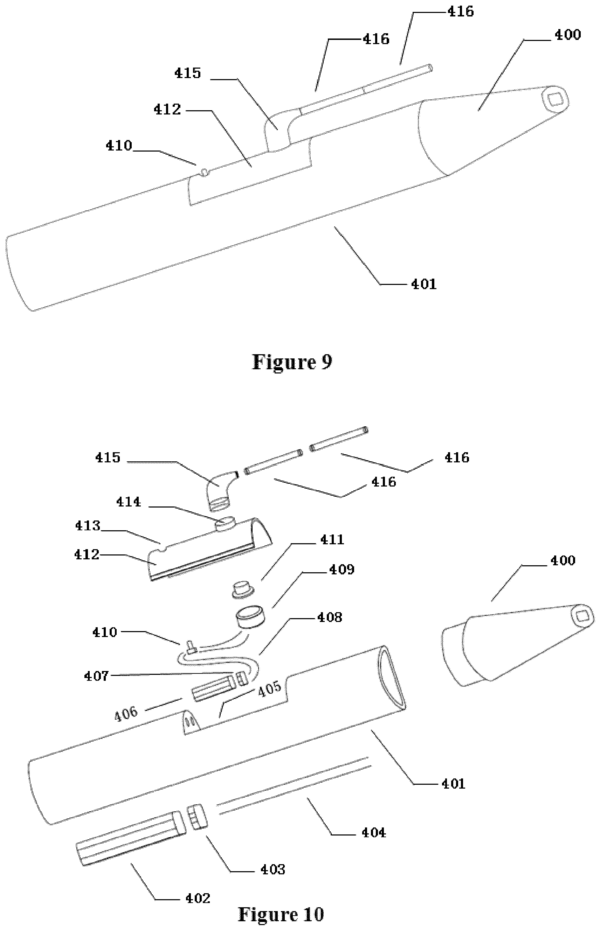

Figure 9 is an external view of the e-cigarette in the Embodiment 4, and Figure 10 is an exploded view of the structure of the e-cigarette in the Embodiment 4, and the components are as follows: 400 atomizing cartridge, 401 cigarette rod, 402 the first battery, 403 the first battery terminal wire interface, 404 atomizer circuit, 405 chamber, 406 the second battery, 407 the second battery terminal wire interface, 408 wires, 409 electric heating pot, 410 electric heating pot knob switch, 411 electric heating pot sealing cover, 412 chamber cover, 413 electric heating pot knob switch cover hole, 414 essence volatiles conduit interface, 415 essence volatiles conduit, 416 conduit extension tube;

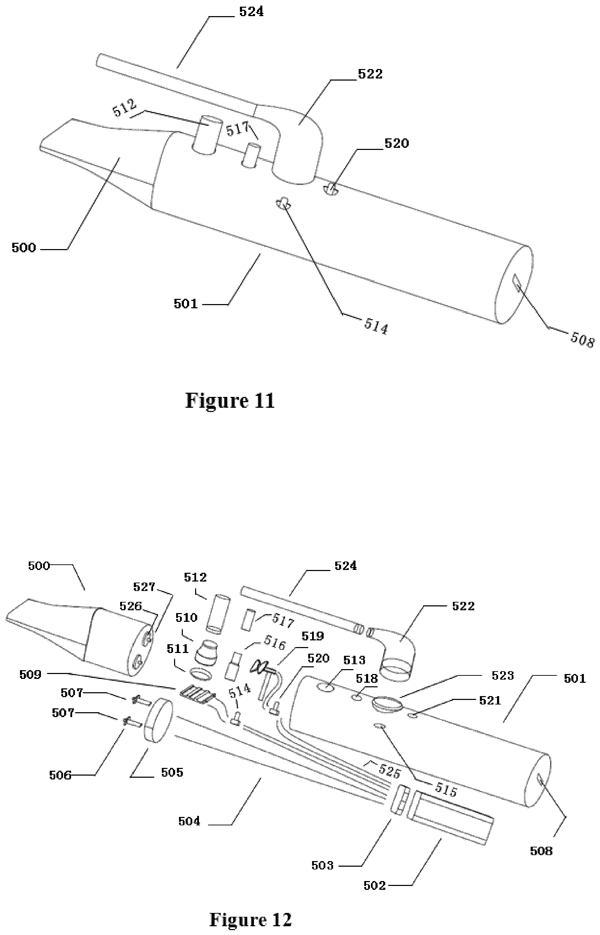

Figure 11 is an external view of the e-cigarette in the Embodiment 5, and Figure 12 is an exploded view of the structure of the e-cigarette in the Embodiment 5, and the components are as follows: 500 atomizing cartridge, 501 cigarette rod, 502 the first battery, 503 the first battery terminal wire interface, 504 atomizer circuit, 505 base, 506 magnetic column in the base, 507 thimble electrode in the base, 508 the first battery charging port, 509 electric heating plate, 510 essence pot, 511 essence pot fixing ring, 512 essence pot spout plug, 513 shell hole for the alignment of essence pot spout plug, 514 electric heating plate knob switch, 515 shell hole for alignment of electric heating plate knob switch, 516 essence bottle, 517 essence bottle cap, 518 shell hole for the alignment of essence bottle cap, 519 fan, 520 fan knob switch, 521 shell hole for the alignment of fan knob switch, 522 essence volatiles conduit, 523 essence volatiles conduit interface, 524 conduit extension tube, 525 wires for electric heating plate and fan, 526 magnetic column in the atomizing cartridge, 527 electrode tip in the atomizing cartridge;

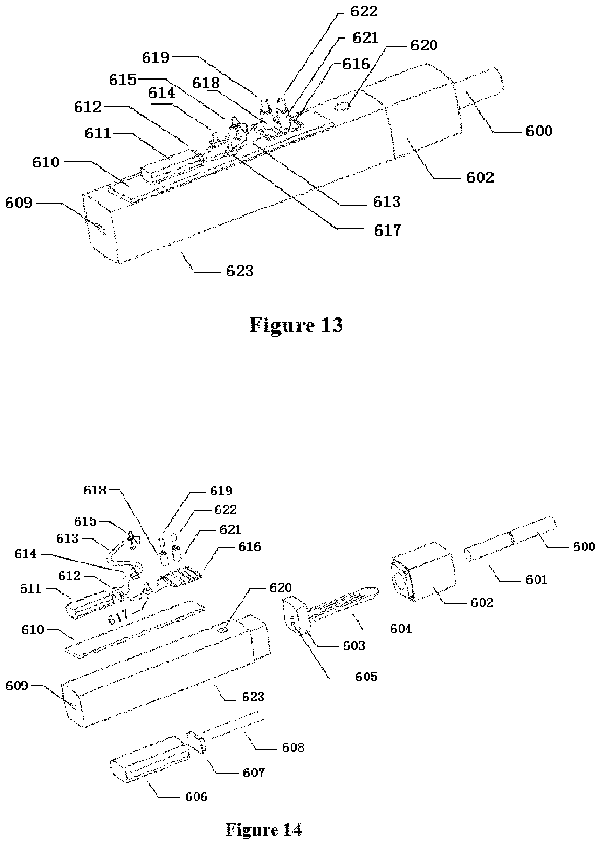

Figure 13 is an external view of the e-cigarette in the Embodiment 6, and Figure 14 is an exploded view of the structure of the e-cigarette in the Embodiment 6, and the components are as follows: 600 filter tip, 601 solid cartridge, 602 cartridge sleeve, 603 heating element base, 604 heating element, 605 heating element electrical connection port, 606 the first battery, 607 the first battery terminal wire interface, 608 heating element circuit, 609 the first battery charging port, 610 double-sided adhesive tape, 611 the second battery, 612 the second battery terminal wire interface, 613 wires, 614 fan knob switch, 615 fan, 616 electric heating plate, 617 electric heating plate knob switch, 618 the first essence bottle, 619 the first essence bottle cap, 620 heating element switch, 621 the second essence bottle, 622 the second essence bottle cap, 623 cigarette rod;

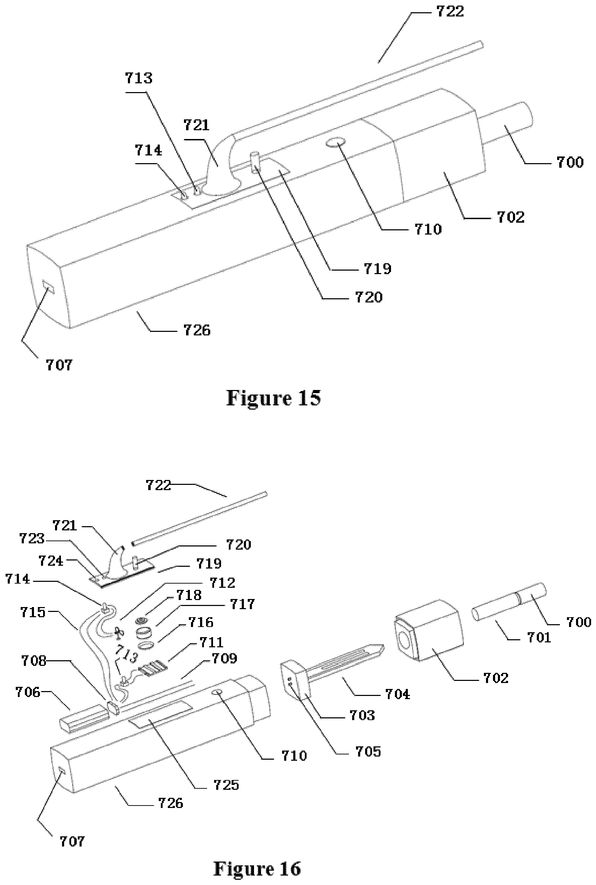

Figure 15 is an external view of the e-cigarette in the Embodiment 7, and Figure 16 is an exploded view of the structure of the e-cigarette in the Embodiment 7, and the components are as follows: 700 filter tip, 701 solid cartridge, 702 cartridge sleeve, 703 heating element base, 704 heating element, 705 heating element electrical connection port, 706 the first battery, 707 the first battery charging port, 708 the first battery terminal wire interface, 709 heating element circuit, 710 heating element switch, 711 electric heating plate, 712 fan, 713 electric heating plate knob switch, 714 fan knob switch, 715 wires, 716 essence pot fixing ring, 717 essence pot, 718 essence pot airtight cover with volatiles outlet valve, 719 chamber cover, 720 push-type switch of the essence pot airtight cover for controlling the volatiles outlet valve, 721 essence volatiles conduit, 722 conduit extension tube, 723 shell hole on the chamber cover for the alignment of electric heating plate knob switch, 724 shell hole on the chamber cover for the alignment of fan knob switch, 725 chamber, 726 cigarette rod.

Remarks, wires are used in the above figures to represent the atomizer circuit and heating element circuit in the atomizing or heating module (12), and these circuits are usually connected with conventional components or electronics used in mouth suction e-cigarette modules such as PCB, starter and sensor.

### Mode of carrying out the Invention

The following will clearly describe the technical solutions in the embodiments of the present invention. Obviously, the described embodiments are only some of the embodiments of the present invention, rather than all the embodiments. Based on the embodiments of the present invention, all other embodiments obtained by person of ordinary skill in the art without making creative effort belong to the protection scope of the present invention.

Referring to Figure 1 - Figure 16 of the accompanying drawings, the embodiments of the present invention include:

### Comparative Example 1

Prepare an ordinary e-liquid and mark it as e-liquid A, and the mass proportion of each component is as follows: 3% benzoate nicotine salt, 2% water, 43% propylene glycol, 39% glycerol, and 1.5% sucralose sweetener, 0.5% neotame sweetener essence, 1% WS-23 cooling agent essence and 10% passion fruit essence, and 2 ml of e-liquid A is injected into an empty atomizing cartridge, forming a set of conventional vapor e-cigarette with a matching cigarette rod, after the atomizing cartridge being inserted into the chamber and connected with the e-cigarette rod, the aerosol generated from the atomization of the e-liquid A can be normally inhaled by mouth.

### Comparative Example 2

A HNB e-cigarette containing tobacco essence in the solid cartridge is purchased from the market, after connecting the solid cartridge, the cartridge sleeve, and the cigarette rod with a sheet-shaped heating element and its base, pushing the circuit switch of the heating element, the smoke generated from the solid cartridge being heated up can be normally inhaled by mouth.

### Embodiment 1: Disposable e-cigarette used by cooperation of mouth and nose, and the essences are partially transferred into the nasal suction module

E-liquid B is prepared by reducing the mass proportion of passion fruit essence and cooling agent essence in e-liquid A. The mass proportions of the components of e-liquid B are as follows: 3% benzoate nicotine salt, 2% water, 43% propylene glycol, 48.5% glycerol, 1.5% sucralose sweetener, 0.5% neotame sweetener essence, 0.7% WS-23 cooling agent essence and 0.8% passion fruit essence. 2 ml of e-liquid B is injected into a disposable vapor e-cigarette set (101), and the mouth suction e-cigarette module, which is composed of the first battery (107), the first battery terminal wire interface (108), atomizer circuit (109), suction nozzle (100) and other components, enables the e-liquid B to be atomized normally, and the user can inhale the atomized aerosol by mouth. As shown in Figure 3 and Figure 4 of the accompanying drawings, two small essence bottles are fixed on the e-cigarette set by a layer of double-sided adhesive tape (106) pre-adhered on the surface of the e-cigarette rod, and 10 drops the above-mentioned passion fruit essence are dropped into the first essence bottle (102), and 10 drops of the above mentioned WS-23 cooling agent essence are dropped into the second essence bottle (104), so as to from a simple nasal suction module (15) constituted with the double-sided adhesive tape, two essence bottles and the contained essences. When using the e-cigarette, the user inhales the atomized aerosol generated from the e-liquid B, choosing one, or opening the first essence bottle cap (103) and the second essence bottle cap (105) at the same time, and inhales the aroma of passion fruit essence and the aroma of cooling agent essence that naturally volatilizing from the two essence bottles, so as to use the e-cigarette by cooperation of mouth and nose.

### Embodiment 2: A cartridge-changing e-cigarette used by cooperation of mouth and nose, and the essences are partially transferred into the nasal suction module

E-liquid C is prepared by removing the cooling agent essence and passion fruit essence from e-liquid A but retaining the sweetener essence. The mass proportions of the components of e-liquid C are as follows: 3% benzoate nicotine salt, 2% water, 43% propylene glycol, 50% glycerol, 1.5% sucralose sweetener, 0.5% neotame sweetener, and 2 ml of the e-liquid C is injected into an empty atomizing cartridge, and the cigarette rod (201) of the cartridge-changing e-cigarette that matched with the interface of the atomizing cartridge is lengthened by 30 mm, after fine-tuning the position of the original circuit module and atomizing or heating module (sensor, PCB board, wires, and the likes) inside of the cigarette rod, an chamber (214) with a length of 30 mm, a width of 15 mm, and a depth of 4 mm in the center is made in the middle of the cigarette rod, and the chamber can be opened or closed by a chamber cover (204) and a chamber cover slot (206) with corresponding size, and the mouth suction e-cigarette module, which is composed of the first battery (210), the first battery terminal wire interface (211), the atomizer circuit (213), the atomizing cartridge (200) and other components, enables the e-liquid C to be atomized normally, and the user can inhale the atomized aerosol by mouth. As shown in Figure 5 and Figure 6 of the accompanying drawings, a miniature essence bottle (202) containing the mixture of an appropriate amount of WS-23 cooling agent essence and passion fruit essence is implanted and fixed into the chamber, as well as a fan (207) aligned with the spout of essence bottle and the user's face; an elongated essence bottle cap (203) is arranged on the essence bottle (202) and extends from the chamber (214) to the outside of the chamber cover (204), a fan knob switch (208) for controlling the opening and rotating speed of the fan is arranged between the chamber and the chamber cover, and an essence bottle cap alignment cover hole (205) and a fan knob switch alignment cover hole (209) are provided on the chamber cover for convenient operation by the user from the outside of the chamber cover. A set of wires (212) are led from the output interface of the first battery to connect with the fan knob switch and the fan; the essence bottle, fan, knob switch and other components then constitute the nasal suction module (15), sharing the first battery with the mouth suction module. When using the e-cigarette, the user inhales the atomized aerosol generated from the e-liquid C by mouth, and at the same time, the user may unscrew the cap of the essence bottle and turn on the fan from the outside of the chamber cover, and inhales the aromas of WS-23 cooling agent essence and passion fruit essence by nose, which are blown out from the essence bottle by the fan, so as to use the e-cigarette by cooperation of mouth and nose.

### Embodiment 3: A cartridge-changing e-cigarette used by cooperation of mouth and nose, and the essences are completely transferred into the nasal suction module

E-liquid D is prepared by removing the cooling agent essence, passion fruit essence and sweetener essence from E-liquid A. The mass proportions of the components of e-liquid D are as follows: 3% benzoate nicotine salt, 2% water, 43% propylene glycol, and 52% glycerol, and 2 ml of the e-liquid D is injected into an empty atomizing cartridge, and the cigarette rod (301) of the cartridge-changing vapor e-cigarette that matched with the interface of the atomizing cartridge is lengthened by 40 mm, after fine-tuning the position of the original circuit module and atomizing or heating module (sensor, PCB board, wires, and the likes) inside of the cigarette rod, an chamber (317) with a length of 40 mm, a width of 15 mm, and a depth of 6 mm in the center is made in the middle of the cigarette rod, and the chamber can be opened or closed by a chamber cover (313) and a chamber cover slot with corresponding size. The mouth suction e-cigarette module, which is composed of the first battery (302), the first battery terminal wire interface (303), the atomizer circuit (304), the atomizing cartridge (300) and other components, enables the e-liquid D to be atomized normally, and the user can inhale the atomized aerosol by mouth. As shown in Figure 7 and Figure 8 of the accompanying drawings, a new lithium-ion battery namely as the second battery (305), is implanted and fixed into the chamber perpendicular to the direction of the cigarette rod, and a charging port (316) is provided for the second battery at the corresponding position of the cigarette rod, two sets of wires (307) are led out through the second battery terminal wire interface (306) to connect a disk-type fan (308) and a mesh electric heating plate (310) in the chamber respectively, a fan controller (309) with knob switch for controlling the fan opening and rotating speed is set between the chamber and the fan controller cover hole (315), and an electric heating plate controller (311) with knob switch for controlling the opening and the temperature level of the mesh electric heating plate is set between the chamber and the electric heating plate controller cover hole (314), which is convenient for users to operate from the outside of the chamber cover; fresh citrus peel as a solid essence (312) is placed on the mesh electric heating plate, and the aroma of citrus peel can be released from the essence aroma outlet (318) preset at the corresponding position of the chamber cover; the second battery, the fan, the electric heating plate, the solid essence citrus peel, the knob switch and other components then constitute the nasal suction module (15). When using the e-cigarette, the user inhales the atomized aerosol generated from the e-liquid D by mouth, and at the same time, the electric heating plate and fan can be controlled from the outside of the chamber cover, and the aroma of the citrus peel as solid essence on the electric heating plate blown out by the fan can be inhaled by nose, so as to use the e-cigarette by cooperation of mouth and nose.

### Embodiment 4: A cartridge-changing e-cigarette used by cooperation of mouth and nose, and all the essences and some of the essence solvent are transferred to the nasal suction module

E-liquid E is prepared by removing the cooling agent essence, passion fruit essence, sweetener essence and essence solvent propylene glycol from e-liquid A. The mass proportions of the components of e-liquid E are as follows: 3% benzoate nicotine salt, 2% water, and 95% glycerol, 2 ml of e-liquid E is injected into an empty atomizing cartridge with self-service injection port (open type), and the cigarette rod (401) of the cartridge-changing vapor e-cigarette that matched with the interface of the atomizing cartridge is lengthened by 10 mm, after fine-tuning the position of the original circuit module and atomizing or heating module (sensor, PCB board, wires, and the likes) inside of the cigarette rod, an chamber (405) with a length of 10 mm, a width of 10 mm, and a depth of 5 mm in the center is made in the middle of the cigarette rod, and the chamber can be opened or closed by a chamber cover (412) and a chamber cover slot with corresponding size. The mouth suction e-cigarette module, which is composed of the first battery (402), the first battery terminal wire interface (403), the atomizer circuit (404), the atomizing cartridge (400) and other components, enables the e-liquid D to be atomized normally, and the user can inhale the atomized aerosol by mouth. As shown in Figure 9 and Figure 10 of the accompanying drawings, a non-rechargeable button battery, as the second battery (406), is implanted and fixed into the chamber, and a set of wires (408) are led out through the second battery terminal wire interface (407) to connect a miniature electric heating pot (409) and an electric heating pot knob switch (410), the electric heating pot is equipped with a resistance heating component; a mixture of ethyl maltol essence powder and pasty mutton fat according to the mass ratio of 3:7 is put into the electric heating pot as the meat essence; the knob switch (410) for controlling the opening and the temperature level of the electric heating pot is set between the chamber cover (412) and the chamber (405), and the knob switch can be operated from the electric heating pot knob switch cover hole (413); an essence volatiles conduit interface (414), an essence volatiles conduit (415) and a conduit extension tube (416) are set in the chamber cover corresponding to the position of the spout of the electric heating pot, which are connected to each other by a thread at the end. The second battery, electric heating pot, the knob switch, essence, essence volatiles conduit assembly and other components then constitute the nasal suction module (15). When using the e-cigarette, the user inhales the atomized aerosol generated from the e-liquid E by mouth, and at the same time, the user can firstly open the chamber cover to take out the sealing cover (411) of the electric heating pot, then close the chamber cover, and then control the knob switch of the heating pot from the outside of the chamber cover, and inhales the meaty aroma mixed with ethyl maltol essence and mutton fat, released from the heating pot and led to nasal by the essence volatiles conduit assembly, so as to use the e-cigarette by cooperation of mouth and nose.

### Embodiment 5: A cartridge-changing e-cigarette used by cooperation of mouth and nose, and the essences and essence solvents are completely transferred to the nasal suction module

E-liquid F is prepared by removing the cooling agent essence, passion fruit essence, sweetener essence, essence solvents propylene glycol and glycerol from e-liquid A. The mass proportions of the components of e-liquid F are 0.3% nicotine and 99.7% water, and 2 ml of e-liquid F is injected into an empty atomizing cartridge without self-service injection port (closed type), and the shell of original cigarette rod is extended by 100 mm to the side of the atomizing cartridge to form a long chamber, and the components of the atomizer circuit (504), the base (505) containing a pair of magnetic columns (506) and a pair of thimble electrodes (507) are moved forward 100 mm in the direction of the atomizing cartridge and correspond to the magnetic column (526) and the electrode tip (527) in the atomizing cartridge respectively. The mouth suction e-cigarette module, which is composed of the atomizing cartridge (500), the extended cigarette rod (501), the sensor and PCB board in the original circuit module, the first battery (502), the first battery terminal wire interface (503), the extension wire and other components, enables the e-liquid F to be atomized normally, and the user can inhale the atomized aerosol by mouth. As shown in Figure 11 and Figure 12 of the accompanying drawings, two sets of wires (525) are led from the first battery terminal wire interface (503) to connect the electric heating plate (509), the electric heating plate knob switch (514), the fan (519) and the fan knob switch (520) respectively, which are set in the chamber. The extended cigarette rod (501) has shell hole (515) and another shell hole (521) corresponding to the positions of the two knob switches respectively, so that the user can control the electric heating plate and the fan from the outside of the cigarette rod. An essence pot fixing ring (511), an essence pot (510), and an elongated essence pot spout plug (512) are sequentially provided on the electric heating plate (509), wherein the essence pot fixing ring is fixed on the electric heating plate by screws, and the essence pot spout plug (512) is extended outside the e-cigarette rod through the alignment shell hole (513), and an appropriate amount of mung bean liquid essence and sweetener neotame essence are added to the essence pot. An essence bottle (516) with a threaded spout is re-implanted and fixed into the chamber, and an elongated essence bottle cap (517) with a corresponding thread is extended outside the cigarette rod through the alignment hole (518) of the cigarette rod shell. The user can open or close the essence pot spout plug and the essence bottle cap through the outside of the e-cigarette rod, and an appropriate amount of rose essence is added into the essence bottle. An opening is provided for the chamber as the essence volatiles conduit interface (523) on the shell of the cigarette rod, and an essence volatiles conduit and a conduit extension tube are arranged, which are connected to each other by threads preset at the ends. The electric heating plate, essence, essence pot, essence pot spout plug, essence bottle, essence bottle cap, fan, knob switch, wire, essence volatiles conduit assembly, and other components then constitute the nasal suction module (15). When using the e-cigarette, the user inhales the atomized aerosol generated from the e-liquid F by mouth, and at the same time, the user can open the essence pot spout plug and the essence bottle cap, so as to release aromas of the mung bean essence, the sweetener essence, and the rose essence from the three outlets, that is the shell hole (513) aligned with the essence pot spout plug, the shell hole (518) aligned with the essence bottle cap, as well as the essence volatiles conduit assembly (523, 522, 524). Two outlets of essence aroma that the shell hole (513) for the alignment of essence pot spout plug and the shell hole (518) for the alignment of essence bottle cap may be covered with fingers, so that the mixed essence aromas can be released from the outlet of the conduit assembly individually, and the speed and temperature at which the essence aroma from the chamber entering the nasal cavity of the user can be adjusted through outside of the cigarette rod by turning on the electric heating plate knob switch (514) and the fan knob switch (520), so as to use the e-cigarette by cooperation of mouth and nose.

### Embodiment 6: HNB e-cigarette used by cooperation of mouth and nose, and the essences are partially transferred into the nasal suction module

An ordinary HNB e-cigarette that using solid cartridge is purchased from the market, wherein components of the mouth suction module mainly include filter tip (600), solid cartridge (601), cartridge sleeve (602), heating element base (603), heating element (604), heating element electrical connection port (605), the first battery (606), the first battery terminal wire interface (607), heating element circuit (608), the first battery charging port (609), heating element switch (620), and the likes, and using a solid cartridge containing only a small amount of essence substances, for example a solid cartridge filled of sheets with a small amount of plant-extracted nicotine and tobacco essence, and the smoke generated from the solid cartridge after being heated by the heating element can be normally inhaled by mouth. As shown in Figure 13 and Figure 14 of the accompanying drawings, a piece of double-sided adhesive tape (610) with a width similar to that of the e-cigarette is adhered on the surface of the e-cigarette set slightly lower than the heating element switch (620), and components such as the second battery (611), the second battery terminal wire interface (612), fan (615), fan knob switch (614), electric heating plate (616) and electric heating plate knob switch (617) are sequentially adhered and fixed on surface of the double-sided adhesive tape, and two sets of wires (613) are led out from the second battery terminal wire interface (612) to connect with the fan, the fan knob switch, the electric heating plate and the electric heating plate knob switch respectively, and two essence bottles are fixed by screws on the electric heating plate, the first essence bottle (618) is provided with an appropriate amount of tobacco essence, and the second essence bottle (621) is provided with some wine essence. The second battery, electric heating plate, fan, essence bottle, knob switch and double-sided adhesive tape then constitute the nasal suction module (15), which is exposed on the HNB e-cigarette rod. When using the e-cigarette, the user inhales the smoke generated from the HNB mouth suction e-cigarette module, and at the same time, the user may open the first essence bottle cap (619) and the second essence bottle cap (622), so that the volatilized essence aromas enter into the user's nasal cavity directly from the two essence bottles, and the user may further turn on and control the fan knob switch as well as the electric heating plate knob switch to adjust the speed and temperature of the essence aromas from the two essence bottles entering into the nasal cavity, so as to use the e-cigarette by cooperation of mouth and nose.

### Embodiment 7: HNB e-cigarettes used by cooperation of mouth and nose, and the essences are completely transferred into the nasal suction module

Extending the shell of the Comparative Example 2 or similar HNB e-cigarette set by 50 mm, and fine-tuning the positions of the original circuit module and atomizing or heating module (PCB board, wires, temperature sensor, and the likes) inside of the HNB e-cigarette rod, an chamber (725) with a length of 50 mm, a width of 12 mm, and a depth of 10 mm in the center is made in the middle of the cigarette rod (726), and the chamber can be opened or closed by a chamber cover (719) and a chamber cover slot with corresponding size. The components of the HNB mouth section e-cigarette module mainly include filter tip (700), solid cartridge (701), cartridge sleeve (702), heating element base (703), heating element (704), heating element electrical connection port (705), the first battery (706), the first battery charging port (707), the first battery terminal wire interface (708), the heating element circuit (709), the heating element switch (710), and the likes, and using solid cartridge without essence substances, for example, smoking pellet cartridge that made with synthetic nicotine or the likes excluding essence, the user can normally inhale the smoke by mouth which is generated from the solid cartridge after being heated by the heating element. As shown in Figure 15 and Figure 16 of the accompanying drawings, a fan (712), an electric heating plate (711), an essence pot fixing ring (716), an essence pot (717) and an essence pot airtight cover with volatiles outlet valve (718) are sequentially implanted and fixed into the chamber, and two sets of wires led from the first battery terminal wire interface (708) connect with the fan (712), the fan knob switch (714), the electric heating plate (711), and the electric heating plate knob switch (713) respectively, and the user operation portions of the two knob switches are fixed on the chamber cover (719) through the shell hole (723) for the alignment of electric heating plate knob switch and the shell hole (724) for the alignment of fan knob switch, which are convenient for the user to control the electric heating plate and the fan from outside the chamber. A push-type switch (720) for connecting the volatiles outlet valve of the airtight cover of the essence pot is arranged on the chamber cover, and the mixture of an appropriate amount of opaque cigarette paste essences of tobacco with tar odor and solid musk essence is added to the essence pot, closing the essence pot airtight cover (718), and the volatiles outlet valve of the essence pot airtight cover can be opened or closed by the push-type switch (720) on the chamber cover (719). The push-type switch (720) and the essence pot airtight cover with volatiles outlet valve, can be further simplified as a carrying rope and an exhaust valve similar to that on an electric cooker airtight cover or on a pressure cooker airtight cover, and the volatiles outlet valve can be opened or closed with the control of the carrying rope.

In this embodiment, an essence volatiles conduit (721) is further provided on the chamber cover (719), which is connected with an conduit extension tube (722) by an end thread. The electric heating plate, the essence pot, the essence pot airtight cover with volatiles outlet valve, the push-type switch of the essence pot airtight cover, the fan, the knob switch, and the volatiles conduit assembly then constitute the nasal suction module (15), which shares the first battery with the mouth suction e-cigarette module. When using the e-cigarette, the user inhales the smoke generated from the HNB mouth suction e-cigarette module by mouth, and at the same time, the push-type switch (720) over the chamber cover can be used to open the volatiles outlet valve of the airtight cover of the essence pot, so that the essence aroma in the essence pot can be led out to the user's nasal cavity by the essence volatiles conduit (721) and the conduit extension tube (722).

### Effects of the present invention:

The nasal suction module is fixed on the shell of e-cigarette set by using double-sided adhesive tape, screws, and the likes in the Embodiment 1 and the Embodiment 6 respectively, and the nasal suction module is implanted into a chamber in the middle of the cigarette rod by appropriately lengthening the shell of e-cigarette set in the Embodiment 2, 3, 4, and 7 respectively, and the nasal suction module is implanted into a properly lengthened chamber of the e-cigarette rod connecting to the atomizing cartridge and the base, magnetic column, electrode, wires are shifted forward accordingly in the Embodiment 5.

The second battery is employed for the electrical appliances of the nasal suction module in the Embodiment 3, 4, and 6, while the first battery in the e-cigarette set is shared by the nasal suction module with the mouth suction module in the Embodiment 2, 5, and 7, and there is no battery for the nasal suction module in the Embodiment 1.

From the Embodiment 1 to the Embodiment 5, the flavor essence, the sweetener essence, the cooling agent essence, the essence solvent propylene glycol, and the essence solvent glycerol are subtracted sequentially from e-liquid A, and the HNB solid cartridge contains only a small amount essence in the Embodiment 6, the HNB solid cartridge does not contain essence ingredient in the Embodiment 7. From the Embodiments of 1-7, the essence and essence solvent are partially or completely transferred from the mouth suction e-cigarette module into the nasal suction module in the present invention, so that the essence substances in the atomized aerosol and smoke entering the lungs from mouth are greatly reduced or even zero, whereby helping users to reduce the risk of essences, essence solvents and essence-related substances entering to the lungs and to improve the using safety of e-cigarette.

In addition, by providing a nasal suction module in the e-cigarette set, the user can directly and quickly inhale the essence aroma, which improves the transmission efficiency of the essence molecules to the user's nasal cavity. Moreover, these essences do not need to undergo the process of being atomized at high temperature together with the e-liquid, but these essence aromas can volatilize and be transmitted to the user's nasal cavity at room temperature, by slightly heated, by fan blown or with volatiles conduit assistance, whereby maintaining the original flavor and physiological activity of the essence substance.

Users can not only continue to inhale the atomized aerosol or smoke generated from the mouth section e-cigarette module by mouth, but also directly inhale the essence aroma released from the nasal section module of e-cigarette by nose, realizing dual suction channels to use the e-cigarette by cooperation of mouth and nose.

Twenty professional users of vapor e-cigarette evaluate the above-mentioned Comparative Example 1 and the Embodiments 1-5 respectively, and the comprehensive opinion shows that the vaping experience of the e-cigarettes used by mouth and nose double suction channels in the Embodiments 1-5 is significantly better than that of used by mouth single suction channel in Comparative Example 1. Specifically, the evaluation is as follows: the Embodiments 1-5 enable the user's nasal cavity to perceive the essence aroma more quickly, improving the transmission efficiency of aroma molecules to the nasal cavity, and because there are not too much interference from other substances, the aroma is more conducive to the rapid identification by the olfactory nerve, improving the flavor reproduction level of e-cigarette.

Twenty professional users of HNB e-cigarette compare the above-mentioned Comparative Example 2 with the Embodiments 6-7 respectively, and the comprehensive opinion is: although the solid cartridges of the Embodiments 6-7 contain little or no tobacco essence, after transferring the tobacco essence and the likes to the nasal suction module, and using the HNB e-cigarette by cooperation of mouth and nose, the tobacco and other essence aromas emitted from the nasal suction module can be sensed and identified by nose more quickly, which improves the transmission efficiency of the aroma to the nasal cavity and increases the available essence types, especially the opaque cigarette paste essences of tobacco with tar odor can be used in the nasal suction module, and the physiological experience of the e-cigarette used by cooperation of mouth and nose is very close to that of traditional flue-cured tobacco.

In addition, the e-cigarette used by cooperation of mouth and nose in the present invention does not limit the smoking material in the mouth section e-cigarette module, wherein the smoking material may contain nicotine and its derivatives such as nicotine salt, and the likes, or it may contain herbal smoking material but without addictive ingredients such as nicotine, and it may further contain health-care or medicinal substances.

The above description are only some embodiments of the present invention, and are not intended to limit the patent scope of the present invention. Any equivalent structure or equivalent process transformation made by using the content of the description of the present invention, and directly or indirectly applied in other related technical fields, shall be all included in the patent protection scope of the present invention.

## Claims

1. An electronic cigarette used by cooperation of mouth and nose, wherein the electronic cigarette comprises a mouth suction electronic cigarette module and a nasal suction module, the mouth suction electronic cigarette module adopts one of a vapor electronic cigarette and a heat-not-burn electronic cigarette, the nasal suction module is arranged outside or inside of the mouth suction electronic cigarette module, and the nasal suction module contains at least one essence carrier, and an essence is placed in the essence carrier.

2. The electronic cigarette used by cooperation of mouth and nose according to claim 1, wherein the vapor electronic cigarette adopts one of a high-power electronic cigarette, a low-power electronic cigarette, a CBD faction electronic cigarette, an open type electronic cigarette, a closed type electronic cigarette, an ultrasonic atomized electronic cigarette, a disposable vapor electronic cigarette, a cartridge-changing vapor electronic cigarette, and the heat-not-burn electronic cigarette adopts one of a heat-not-burn electronic cigarette that consuming solid cartridge and a heat-not-burn electronic cigarette that consuming cigarette paste.

3. The electronic cigarette used by cooperation of mouth and nose according to claim 1, wherein the essence is a combination of one or more of fruit peel, fruit, fruit essence, cooling agent essence, sweetener essence, tobacco essence, meat essence, wine essence, food essence, plant herbal essence, animal spice essence, biosynthetic essence and microbial fermentation essence.

4. The electronic cigarette used by cooperation of mouth and nose according to claim 1, wherein the electronic cigarette further comprises a component retainer for fixing the nasal suction module outside or inside of the mouth suction electronic cigarette module, the component retainer including but not limited of double-sided adhesive tape, gummed tape, glue, string, clamp, snap hook, magnet, screw, and fixing ring.

5. The electronic cigarette used by cooperation of mouth and nose according to claim 1, wherein the essence carrier adopts a combination of one or more of essence bottle, essence pot, electric heating plate and electric heating pot, and the electric heating plate adopts a miniature mesh electric heating plate or a non-mesh electric heating plate with integrated resistive heating component.

6. The electronic cigarette used by cooperation of mouth and nose according to claim 1, wherein a first battery is arranged in the mouth suction electronic cigarette module, and the nasal suction module shares the first battery with the mouth suction electronic cigarette module, or the nasal suction module contains a second battery or without battery.

7. The electronic cigarette used by cooperation of mouth and nose according to claim 6, wherein the nasal suction module further contains an electric heater controller, and the first battery or the second battery is connected with the electric heater controller for power supply, and the electric heater controller is connected with the heating component in the essence carrier for heating control.

8. The electronic cigarette used by cooperation of mouth and nose according to claim 6, wherein the nasal suction module further comprises a fan controller and a fan corresponding to the essence carrier, and the first battery or the second battery is connected with the fan controller for power supply, and the fan controller is connected with the fan for operation control.

9. The electronic cigarette used by cooperation of mouth and nose according to claim 1, wherein a chamber corresponding to the nasal suction module is set in the shell of the mouth suction electronic cigarette module, and the nasal suction module is implanted into the chamber.

10. The electronic cigarette used by cooperation of mouth and nose according to claim 1, wherein the nasal suction module further comprises a volatiles conduit, and one end of the volatiles conduit is close to the essence carrier or is directly or indirectly connected with the essence carrier.
